# EUROPEAN PATENT SPECIFICATION

(11) **EP 3 888 741 B1**
(45) Date of publication and mention of the grant of the patent: **17.05.2023**
(21) Application number: 20213738.6
(22) Date of filing: 14.12.2020
(51) Int. Cl.: A61N 1/39

(54) **DEFIBRILLATOR ASSESSING CHEST RECOIL IN CARDIO PULMONARY RESUSCITATION**
DEFIBRILLATOR ZUR BEURTEILUNG DER BRUSTENTLASTUNG BEI DER HERZ-LUNGEN-WIEDERBELEBUNG
DÉFIBRILLATEUR ÉVALUANT LA DÉTENTE THORACIQUE EN RÉANIMATION CARDIO-PULMONAIRE

(30) Priority: 03.04.2020 GB 202004936
(43) Date of publication of application: 06.10.2021
(73) Proprietor: HeartSine Technologies Limited, Belfast Antrim BT3 9ED (GB)
(72) Inventor: McAlister, Olibhear, Belfast, BT3 9ED (GB); Harvey, Adam, Belfast, BT3 9ED (GB); Donaghy, Dymphna, Belfast, BT3 9ED (GB); Anderson, Johnny, Belfast, BT3 9ED (GB)
(74) Representative: HGF

(56) References cited:
- WO-A1-2010/009531
- US-A1- 2017 143 584
- US-A1- 2018 169 426
- US-A1- 2019 008 720
- US-B2- 10 512 749

## Description

The invention relates to a defibrillator which assesses chest recoil of a subject during performance of cardio pulmonary resuscitation (CPR) by a rescuer on the subject.

Various circumstances may arise when a defibrillator instructs a rescuer to carry out CPR on a subject being treated. CPR involves multiple compressions of the subject's chest by the rescuer to cause the subject's heart to pump blood around their circulatory system, primarily to provide oxygenated blood to the subject's heart and brain. It is important that, following a chest compression, the rescuer allows the subject's chest to completely recoil, i.e. return to its normal position, before the rescuer begins the next chest compression. If this not the case, the subject's heart will not completely refill with blood, the heart will not pump sufficient oxygenated blood and the heart and brain tissue will start to die. For effective CPR, current guidelines recommend complete recoil of a subject's chest. Indeed, the European Resuscitation Council Guidelines for adult basic life support and automated external defibrillation state that a rescuer should : *After each compression allow the chest to recoil completely.* However, many rescuers do not allow complete recoil of the chest. Defibrillators which assess, and provide feedback concerning, the subject's chest recoil could improve the quality of CPR provided by a rescuer.

US 2019/008720 discloses an automated chest compression (CC) system that includes a chest compressor configured to administer chest compressions to a patient, at least one tilt adjuster configured to tilt at least the head of the patient to a tilt angle during the administration of chest compressions to the patient, a patient support structure configured to couple to the chest compressor and to the at least one tilt adjuster, one or more tilt sensors, and a CC device controller configured to control the chest compressor to administer the chest compressions at a resuscitative rate, receive one or more signals, from the one or more tilt sensors, indicative of the tilt angle, determine tilt angle information from the one or more signals indicative of the tilt angle, and provide the tiltangle information to a user interface, wherein the patient support structure is adapted to support the back of the patient.

WO 2010/009531 discloses a device for the determination of at least one compression parameter during administration of cardiopulmonary resuscitation (CPR) on a patient comprising: a field generator, a field detector, and a processor. Position information and the compression parameter are determined from the field detected by the field detector. One of the field generator and the field detector is a position sensor and the other is a reference sensor.

US 2017/143584 discloses a method and device for the determination and calculation of the depth of chest compressions during the administration of cardiopulmonary resuscitation (CPR). Embodiments use an optical sensor to monitor the distance that a victim's chest is displaced during each compression throughout the administration of CPR. The optical sensor is most commonly an image sensor such as a CMOS or CCD sensor, and more specifically a CMOS image sensor capable of three-dimensional imaging based on the time-of-flight principle. An infrared emitter may illuminate the victim's body and any visible piece of ground beside the victim. As the infrared light interacts with any surfaces it encounters, it is reflected and returns to the image sensor where the time of flight ofthe infrared light is calculated for every pixel in the image sensor. The distance data is used to gauge the effective displacement of the victim's chest. The optical sensors can be used to visualize the size of a patient and immediately gauge the body type and instruct the user accordingly. Furthermore, optical measurement techniques can be used to accurately measure chest rise during artificial respiration and ensure that proper ventilation is being administered in between compressions. In addition, optical measurements of the chest of the victim and the hands of the rescuer can be used to help ensure that the rescuer has positioned his or her hands in the anatomically correct location for effective CPR.

US 2018/169426 discloses a device, and software and methodology associated with a portable Automated External Defibrillator ("AED"). The portable AED works with a mobile device and software, and includes two or more cardiac pads, a battery pack, and specialized capacitor. When connected to a patient in cardiac arrest, the AED contacts Emergency Medical Services, and records patient information to be transmitted for evaluation by medical providers. The AED is able to analyze cardiac rhythms, suggests administering one or more shocks to the patient in appropriate cardiac arrhythmia, and guides a user on proper CPR technique, if enabled. The AED software can alert other personnel via a mobile device app.

US 10512749 discloses medical techniques including systems and methods for administering a positive pressure ventilation, a positive end expiratory pressure, and a vacuum to a person. Approaches also include treating a person with an intrathoracic pressure regulator so as to modulate or upregulate the autonomic system of the person, and treating a person with a combination of an intrathoracic pressure regulation treatment and an intra-aortic balloon pump treatment.

The invention is defined in the appended claims. Aspects, embodiments and examples disclosed herein which do not fall within the scope of the appended claims do not form part of the invention, and are merely provided for illustrative purposes.

According to the invention there is provided a defibrillator which assesses chest recoil of a subject during cardio pulmonary resuscitation (CPR) carried out by a rescuer on the subject and provides feedback to the rescuer, comprising:
a biosignal measurement system configured to measure biosignals of the subject, determine when CPR is required and produce a CPR start signal and determine when CPR is to be ceased and produce a CPR stop signal,
an impedance measurement system configured to measure impedance signals of the subject,
a CPR assessment system connected to the biosignal measurement system to receive the CPR start signal and the CPR stop signal, connected to the impedance measurement system to receive impedance signals and configured to perform the steps:
   (i) receive the CPR start signal and produce a first feedback signal,
   (ii) receive an impedance signal of the subject measured during a plurality of CPR chest compressions by the rescuer,
   (iii) use the impedance signal to assess chest recoil of the subject during the plurality of CPR chest compressions,
   (iv) compare the chest recoil of the subject with a chest recoil threshold,
   (v) when the chest recoil of the subject is greaterthan the chest recoil threshold, produce a second feedback signal and go to step (vii),
   (vi) when the chest recoil of the subject is less than the chest recoil threshold, produce a third feedback signal and go to step (vii),
   (vii) when the CPR stop signal is not received, return to step (ii),
   (viii) when the CPR stop signal is received, produce a fourth feedback signal,
   and
a feedback unit connected to the CPR assessment system and configured to receive the feedback signals and issue CPR feedback to the rescuer.

The aim is to achieve as close to complete chest recoil as possible after each CPR chest compression and the defibrillator provides appropriate feedback to the rescuer to try to achieve this.

The CPR assessment system may use the impedance signal to assess chest recoil of the subject during the plurality of CPR chest compressions by measuring a characteristic of the impedance signal after at least some of the plurality of CPR chest compressions. The CPR assessment system may use the impedance signal to assess chest recoil of the subject during the plurality of CPR chest compressions by measuring an amplitude of the impedance signal after at least some of the plurality of CPR chest compressions.

The CPR assessment system may compare the amplitude of the impedance signal after at least some of the plurality of CPR chest compressions with an impedance baseline of the subject. The impedance signal may comprise a series of peaks and troughs corresponding to the plurality of CPR chest compressions by the rescuer. The CPR assessment system may compare the amplitude of a trough of the impedance signal after at least some of the plurality of CPR chest compressions with the impedance baseline of the subject. The CPR assessment system may compare the amplitude of a peak of the impedance signal after at least some of the plurality of CPR chest compressions with the impedance baseline of the subject.

The CPR assessment system may determine either incomplete chest recoil when the amplitude of the impedance signal after a CPR chest compression is not equal to the impedance baseline or complete chest recoil when the amplitude of the impedance signal after a CPR chest compression is equal to the impedance baseline for at least some of the plurality of CPR chest compressions. The CPR assessment system may determine either incomplete chest recoil when the amplitude of the impedance signal after a CPR chest compression is not within a predetermined tolerance of the impedance baseline or complete chest recoil when the amplitude of the impedance signal after a CPR chest compression is within a predetermined tolerance of the impedance baseline for at least some of the plurality of CPR chest compressions. The predetermined tolerance may be 10% of the amplitude of the impedance signal after a CPR chest compression.

The CPR assessment system may determine a proportion of incomplete chest recoils for the at least some of the plurality of CPR chest compressions. The CPR assessment system may compare the chest recoil of the subject with the chest recoil threshold by comparing the proportion of incomplete chest recoils with the chest recoil threshold. The CPR assessment system may produce the second feedback signal when the proportion of incomplete chest recoils is greater than the chest recoil threshold. The CPR assessment system may produce the third feedback signal when the proportion of incomplete chest recoils is less than the chest recoil threshold. The chest recoil threshold may be a proportion of incomplete chest recoils of 25%.

The impedance baseline may be established during at least one period in which no CPR chest compressions are performed by the rescuer. The impedance measurement system may be configured to measure impedance signals during at least one period in which no CPR chest compressions are performed by the rescuer. The CPR assessment system may be configured to receive an impedance signal of the subject measured during the at least one period in which no CPR chest compressions are performed by the rescuer and use the impedance signal to establish the impedance baseline of the subject. The CPR assessment system may use an amplitude of the impedance signal measured during the at least one period to establish the impedance baseline of the subject. The CPR assessment system may be configured to receive an impedance signal of the subject measured during a period before CPR chest compressions by the rescuer and use the impedance signal to establish the impedance baseline. The CPR assessment system may be configured to receive an impedance signal of the subject measured during one or more periods after CPR chest compressions by the rescuer and use the impedance signal to establish one or more impedance baselines. The CPR assessment system may be configured to receive an impedance signal of the subject measured during a first period before CPR chest compressions by the rescuer and use the impedance signal to establish a first impedance baseline and receive an impedance signal of the subject measured during one or more subsequent periods after CPR chest compressions by the rescuer and use the impedance signal to establish one or more subsequent impedance baselines.

The CPR assessment system may receive an impedance signal of the subject measured during the plurality of CPR chest compressions by the rescuer over a period of time of approximately 6 seconds. The CPR assessment system may receive an impedance signal during a period in which no CPR chest compressions are performed by the rescuer over a period of time of approximately 2 seconds.

The CPR assessment system may be configured to assess rate and depth of at least some of the plurality of CPR chest compressions by the rescuer.

The impedance measurement system may measure the impedance signals of the subject by acquiring signals through electrodes of the defibrillator placed on the chest of the subject.

The biosignal measurement system may be configured to measure biosignals of the subject in the form of ECG biosignals. The biosignal measurement system may use an algorithm on the ECG biosignals to determine if the subject is exhibiting a condition which requires a defibrillation shock or a condition which requires CPR.

Steps (v) to (vii) may further comprise:
(v) when the chest recoil of the subject is greaterthan the chest recoil threshold, produce the second feedback signal and go to step (vii)(a),
(vi) when the chest recoil of the subject is less than the chest recoil threshold, produce the third feedback signal and go to step (vii)(b),
(vii)(a) when the CPR stop signal is not received, wait for a plurality of CPR chest compressions, return to step (ii),
(vii)(b) when the CPR stop signal is not received, return to step (ii).

Step (ii) may further comprise:
(ii)(a) set a CPR counter equal to x,
(ii)(b) receive an impedance signal of the subject measured during a plurality of CPR chest compressions by the rescuer,
(ii)(c) receive a CPR chest compression rate measured during the plurality of CPR chest compressions,
(ii)(d) compare the measured compression rate with a minimum required compression rate,
(ii)(e) when the measured compression rate is greater than the minimum required compression rate, go to step (iii),
(ii)(f) when the measured compression rate is less than the minimum required compression rate, when the CPR counter is not equal to zero, decrease the CPR counter by 1, produce a fifth feedback signal and go to step (ii)(b),
(ii)(g) when the measured compression rate is less than the minimum required compression rate, when the CPR counter is equal to zero, go to step (iii).

Step (ii)(f) may further comprise when the measured compression rate is less than the minimum required compression rate, when the CPR counter is not equal to zero, decrease the CPR counter by 1, produce a fifth feedback signal, wait for a plurality of compressions and go to step (ii)(b).

Alternatively, step (ii) may further comprise:
(ii)(a) set a CPR counter equal to x,
(ii)(b) receive an impedance signal of the subject measured during a plurality of CPR chest compressions by the rescuer,
(ii)(c) receive a compression rate measured during the plurality of CPR chest compressions,
(ii)(d) compare the measured compression rate with a maximum required compression rate,
(ii)(e) when the measured compression rate is less than the maximum required compression rate, go to step (iii),
(ii)(f) when the measured compression rate is greater than the maximum required compression rate, when the CPR counter is not equal to zero, decrease the CPR counter by 1, produce a sixth feedback signal and go to step (ii)(b),
(ii)(g) when the measured compression rate is greater than the maximum required compression rate, when the CPR counter is equal to zero, go to step (iii).

Step (ii)(f) may further comprise when the measured compression rate is greater than the maximum required compression rate, when the CPR counter is not equal to zero, decrease the CPR counter by 1, produce a sixth feedback signal, wait for a plurality of compressions and go to step (ii)(b).

Alternatively, step (ii) may further comprise:
(ii)(a) set a CPR counter equal to x,
(ii)(b) receive an impedance signal of the subject measured during a plurality of CPR chest compressions by the rescuer,
(ii)(c) receive a compression rate measured during the plurality of CPR chest compressions,
(ii)(d) compare the measured compression rate with a minimum required compression rate and a maximum required compression rate,
(ii)(e) when the measured compression rate is greater than the minimum required compression rate and when the measured compression rate is less than the maximum required compression rate, go to step (iii),
(ii)(f) when the measured compression rate is less than the minimum required compression rate, when the CPR counter is not equal to zero, decrease the CPR counter by 1, produce a fifth feedback signal and go to step (ii)(b),
(ii)(g) when the measured compression rate is less than the minimum required compression rate, when the CPR counter is equal to zero, go to step (iii), (ii)(h) when the measured compression rate is greater than the maximum required compression rate, when the CPR counter is not equal to zero, decrease the CPR counter by 1, produce a sixth feedback signal and go to step (ii)(b),
(ii)(i) when the measured compression rate is greater than the maximum required compression rate, when the CPR counter is equal to zero, go to step (iii).

Step (ii)(f) may further comprise when the measured compression rate is less than the minimum required compression rate, when the CPR counter is not equal to zero, decrease the CPR counter by 1, produce a fifth feedback signal, wait for a plurality of compressions and go to step (ii)(b).

Step (ii)(h) may further comprise when the measured compression rate is greater than the maximum required compression rate, when the CPR counter is not equal to zero, decrease the CPR counter by 1, produce a sixth feedback signal, wait for a plurality of compressions and go to step (ii)(b).

Step (ii) may be further comprise:
(ii)(a) set a CPR counter equal to x,
(ii)(b) receive an impedance signal of the subject measured during a plurality of CPR chest compressions by the rescuer,
(ii)(c) receive a CPR chest compression depth measured during the plurality of CPR chest compressions,
(ii)(d) compare the measured compression depth with a minimum required compression depth,
(ii)(e) when the measured compression depth is greater than the minimum required compression depth, go to step (iii),
(ii)(f) when the measured compression depth is less than the minimum required compression depth, when the CPR counter is not equal to zero, decrease the CPR counter by 1, produce a seventh feedback signal and go to step (ii)(b),
(ii)(g) when the measured compression rate is less than the minimum required compression rate, when the CPR counter is equal to zero, go to step (iii).

Step (ii)(f) may further comprise when the measured compression depth is less than the minimum required compression depth, when the CPR counter is not equal to zero, decrease the CPR counter by 1, produce a seventh feedback signal, wait for a plurality of compressions and go to step (ii)(b).

Alternatively, step (ii) may further comprise:
(ii)(a) set a CPR counter equal to x,
(ii)(b) receive an impedance signal of the subject measured during a plurality of CPR chest compressions by the rescuer,
(ii)(c) receive a compression depth measured during the plurality of CPR chest compressions,
(ii)(d) compare the measured compression depth with a maximum required compression depth,
(ii)(e) when the measured compression depth is less than the maximum required compression depth, go to step (iii),
(ii)(f) when the measured compression depth is greater than the maximum required compression depth, when the CPR counter is not equal to zero, decrease the CPR counter by 1, produce an eighth feedback signal and go to step (ii)(b),
(ii)(g) when the measured compression depth is greater than the maximum required compression depth when the CPR counter is equal to zero, go to step (iii).

Step (ii)(f) may further comprise when the measured compression depth is greater than the maximum required compression depth, when the CPR counter is not equal to zero, decrease the CPR counter by 1, produce an eighth feedback signal, wait for a plurality of compressions and go to step (ii)(b).

Alternatively, step (ii) may further comprise:
(ii)(a) set a CPR counter equal to x,
(ii)(b) receive an impedance signal of the subject measured during a plurality of CPR chest compressions by the rescuer,
(ii)(c) receive a compression depth measured during the plurality of CPR chest compressions,
(ii)(d) compare the measured compression depth with a minimum required compression depth and a maximum required compression depth,
(ii)(e) when the measured compression depth is greater than the minimum required compression depth and when the measured compression depth is less than the maximum required compression depth, go to step (iii),
(ii)(f) when the measured compression depth is less than the minimum required compression depth, when the CPR counter is not equal to zero, decrease the CPR counter by 1, produce a seventh feedback signal and go to step (ii)(b),
(ii)(g) when the measured compression depth is less than the minimum required compression depth, when the CPR counter is equal to zero, go to step (iii), (ii)(h) when the measured compression depth is greater than the maximum required compression depth, when the CPR counter is not equal to zero, decrease the CPR counter by 1, produce an eighth feedback signal and go to step (ii)(b),
(ii)(i) when the measured compression depth is greater than the maximum required compression depth, when the CPR counter is equal to zero, go to step (iii).

Step (ii)(f) may further comprise when the measured compression depth is less than the minimum required compression depth, when the CPR counter is not equal to zero, decrease the CPR counter by 1, produce a seventh feedback signal, wait for a plurality of compressions and go to step (ii)(b).

Step (ii)(h) may further comprise when the measured compression depth is greater than the maximum required compression depth, when the CPR counter is not equal to zero, decrease the CPR counter by 1, produce an eighth feedback signal, wait for a plurality of compressions and go to step (ii)(b).

The feedback unit may receive the first feedback signal and issue CPR feedback in the form of a 'Start CPR and Push Hard' instruction to the rescuer. The feedback unit may receive the second feedback signal and issue CPR feedback in the form of a 'Chest Recoil Incomplete' instruction to the rescuer. The feedback unit may receive the third feedback signal and issue CPR feedback in the form of a 'Chest Recoil Good' instruction to the rescuer. The feedback unit may receive the fourth feedback signal and issue CPR feedback in the form of a 'Stop CPR' instruction to the rescuer. The feedback unit may receive the fifth feedback signal and issue CPR feedback in the form of a 'Push Faster' instruction to the rescuer. The feedback unit may receive the sixth feedback signal and issue CPR feedback in the form of a 'Push Slower' instruction to the rescuer. The feedback unit may receive the seventh feedback signal and issue CPR feedback in the form of a 'Push Harder' instruction to the rescuer. The feedback unit may receive the eighth feedback signal and issue CPR feedback in the form of a 'Push Softer' instruction to the rescuer.

An embodiment of the invention will now be described, by way of example only, with reference to the following drawings, in which :
Figure 1 is a schematic representation of a defibrillator according to the invention, and
Figure 2 is a flowchart of steps carried out by a CPR assessment system of the defibrillator of Figure 1.

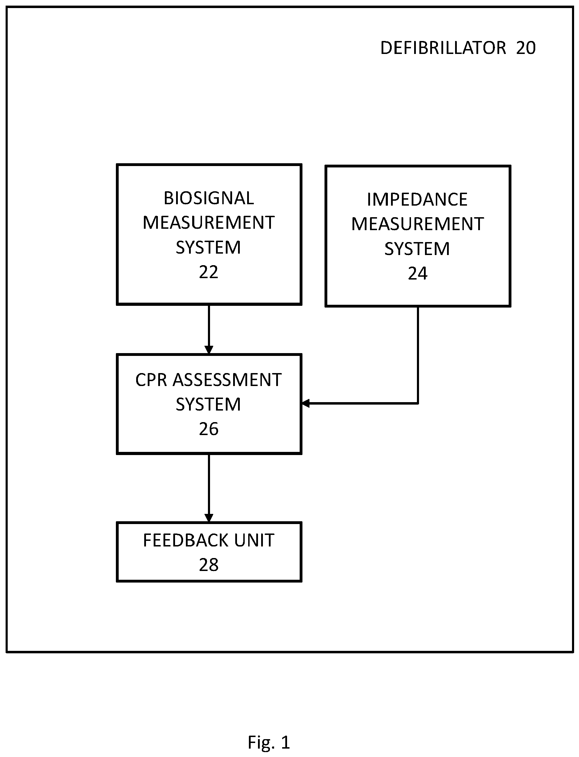

Referring to Figure 1, the defibrillator 20 comprises a biosignal measurement system 22, an impedance measurement system 24, a CPR assessment system 26 and a feedback unit 28. The defibrillator 20 assesses CPR carried out by a rescuer (not shown) on a subject (not shown) to assess chest recoil of the subject and provides CPR feedback to the rescuer.

It will be appreciated that the defibrillator 20 will comprise other elements such as an activation mechanism, a biosignal processing system, defibrillation shock generation circuitry, a power source and a sensing unit which is adapted to be attached to the subject.

The biosignal measurement system 22 is configured to measure biosignals of the subject, in this embodiment, ECG biosignals. An algorithm uses the ECG biosignals to determine if the subject is exhibiting a condition which requires a defibrillation shock or a condition which requires CPR. When CPR is required, the biosignal measurement system 22 is configured to produce a CPR start signal. When CPR is to be ceased, the biosignal measurement system 22 is configured to produce a CPR stop signal.

The impedance measurement system 24 measures the impedance signals of the subject by acquiring signals through electrodes of the defibrillator placed on the chest of the subject.

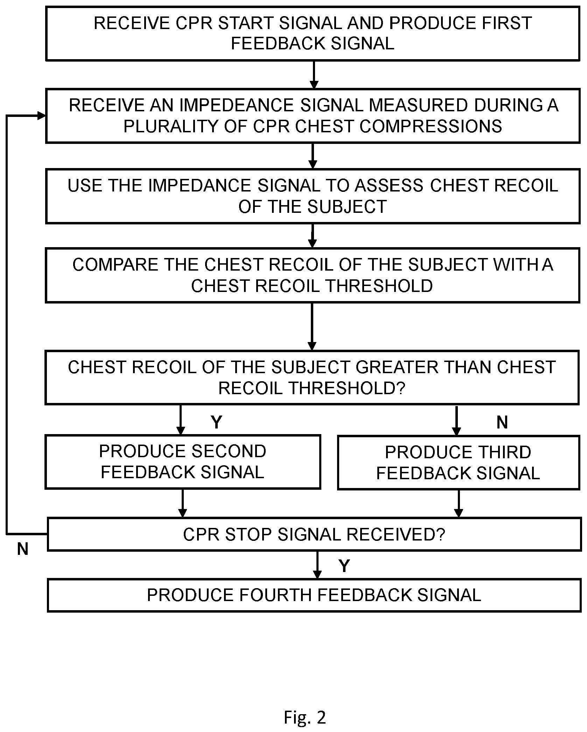

The CPR assessment system 26 is connected to the biosignal measurement system 22 to receive the CPR start signal and the CPR stop signal. On receipt of the CPR start signal, the CPR assessment system 26 receives impedance signals and commences assessment of chest recoil of the subject over multiple pluralities of CPR chest compressions by the rescuer. This comprises performance of a number of steps, described below with reference to Figure 2. On receipt of the CPR stop signal, the CPR assessment system 26 ceases assessment of chest recoil of the subject.

The CPR assessment system 26 is connected to the impedance measurement system 24 and receives impedance signals indicative of CPR chest compressions comprising transthoracic impedance signals and uses these to assess chest recoil of the subject over multiple pluralities of CPR chest compressions carried out by the rescuer on the subject.

During assessment of the chest recoil of the subject, the CPR assessment system 26 produces various feedback signals. The feedback unit 28 is connected to the CPR assessment system 26 and is configured to receive the feedback signals and issue CPR feedback to the rescuer.

Referring to Figure 2, the steps performed by the CPR assessment system 26 of the defibrillator 20 of Figure 1 will be described.

On receipt of the CPR start signal, the CPR assessment system 26 produces a first feedback signal. This is received by the feedback unit 28, which issues CPR feedback in the form of a 'Start CPR and Push Hard' instruction, to the rescuer carrying out CPR on the subject.

The CPR assessment system 26 receives an impedance signal of the subject measured during a plurality of CPR chest compressions by the rescuer over a period of time of approximately 6 seconds and uses the impedance signal to assess chest recoil of the subject during the plurality of CPR chest compressions. The CPR assessment system 26 uses the impedance signal to assess chest recoil by measuring a characteristic of the impedance signal, comprising an amplitude, after at least some, preferably each, of the plurality of CPR chest compressions.

The CPR assessment system 26 then compares the amplitude of the impedance signal after at least some, preferably each, of the plurality of CPR chest compressions with an impedance baseline of the subject. The impedance signal of the subject may comprise a series of peaks and troughs corresponding to the plurality of CPR chest compressions by the rescuer and the CPR assessment system 26 may compare the amplitude of a trough or a peak of the impedance signal after at least some, preferably each, of the plurality of CPR chest compressions with the impedance baseline of the subject.

The CPR assessment system 26 then determines either incomplete chest recoil when the amplitude of the impedance signal after a CPR chest compression is not equal to the impedance baseline or complete chest recoil when the amplitude of the impedance signal after a CPR chest compression is equal to the impedance baseline. It will be appreciated that the CPR assessment system 26 may determine either incomplete chest recoil when the amplitude of the impedance signal after a CPR chest compression is not is not within a predetermined tolerance of the impedance baseline or complete chest recoil when the amplitude of the impedance signal after a CPR chest compression is not within a predetermined tolerance of the impedance baseline. The predetermined tolerance may be approximately 10% of the amplitude of the impedance signal after a CPR chest compression.

The CPR assessment system 26 determines a proportion of incomplete chest recoils and compares the chest recoil of the subject with a chest recoil threshold by comparing the proportion of incomplete chest recoils with the chest recoil threshold, which may be a proportion of incomplete chest recoils of 25%. The CPR assessment system 26 produces a second feedback signal when the proportion of incomplete chest recoils is greater than the chest recoil threshold. This is received by the feedback unit 28, which issues CPR feedback, in the form of a 'Chest Recoil Incomplete' instruction, to the rescuer carrying out CPR on the subject. Alternatively, the CPR assessment system 26 produces the third feedback signal when the proportion of incomplete chest recoils is less than the chest recoil threshold. This is received by the feedback unit 28, which issues CPR feedback in the form of a 'Chest Recoil Good' instruction, to the rescuer carrying out CPR on the subject.

The CPR assessment system 26 then checks for receipt of the CPR stop signal from the biosignal measurement system 22. When the CPR stop signal has not been received, the CPR assessment system 26 returns to the step of receiving an impedance signal during a plurality of CPR chest compressions by the rescuer. When the CPR stop signal is received, the CPR assessment system 26 produces a fourth feedback signal. The feedback unit 28 receives the fourth feedback signal and issues CPR feedback in the form of a 'Stop CPR' instruction to the rescuer carrying out CPR on the subject.

To determine the impedance baseline of the subject, the impedance measurement system 24 is configured to measure impedance signals during at least one period in which no CPR chest compressions are performed by the rescuer. The CPR assessment system 26 is configured to receive an impedance signal of the subject measured during the at least one period in which no CPR chest compressions are performed by the rescuer and use the impedance signal to establish the impedance baseline of the subject. In a preferred embodiment, the CPR assessment system 26 is configured to receive an impedance signal of the subject measured during a first period before CPR chest compressions by the rescuer and use the impedance signal to establish a first impedance baseline and receive an impedance signal of the subject measured during one or more subsequent periods after CPR chest compressions by the rescuer and use the impedance signal to establish one or more subsequent impedance baselines. Each subsequent impedance baseline replaces a previous impedance baseline. The first period is when the defibrillator 20 is making a decision as to whether the subject is exhibiting a condition which requires a defibrillation shock or a condition which requires CPR. The one or more subsequent periods are when the defibrillator 20 instructs the rescuerto cease CPR chest compressions.

The CPR assessment system 26 receives an impedance signal of the subject measured during the period in which no CPR chest compressions are performed by the rescuer and uses an amplitude of the impedance signal to establish the impedance baseline of the subject. When the subject is experiencing a condition which requires a defibrillation shock, i.e. ventricular fibrillation or ventricular tachycardia, the impedance signal of the subject measured during the period in which no CPR chest compressions are performed by the rescuer is a substantially flat line.

The aim is to achieve as close to complete chest recoil as possible after each CPR chest compression and the defibrillator provides appropriate feedback to the rescuer to try to achieve this.

Steps performed by the CPR assessment system 26 may further comprise the following.

Steps (v) to (vii) may further comprise:
(v) when the chest recoil of the subject is greaterthan the chest recoil threshold, produce the second feedback signal and go to step (vii)(a),
(vi) when the chest recoil of the subject is less than the chest recoil threshold, produce the third feedback signal and go to step (vii)(b),
(vii)(a) when the CPR stop signal is not received, wait for a plurality of CPR chest compressions, return to step (ii),
(vii)(b) when the CPR stop signal is not received, return to step (ii).

The CPR assessment system 26 may be configured to assess rate and depth of at least some of a plurality of CPR chest compressions by the rescuer.

Step (ii) may further comprise:
(ii)(a) set a CPR counter equal to x,
(ii)(b) receive an impedance signal of the subject measured during a plurality of CPR chest compressions by the rescuer,
(ii)(c) receive a CPR chest compression rate measured during the plurality of CPR chest compressions,
(ii)(d) compare the measured compression rate with a minimum required compression rate,
(ii)(e) when the measured compression rate is greater than the minimum required compression rate, go to step (iii),
(ii)(f) when the measured compression rate is less than the minimum required compression rate, when the CPR counter is not equal to zero, decrease the CPR counter by 1, produce a fifth feedback signal and go to step (ii)(b),
(ii)(g) when the measured compression rate is less than the minimum required compression rate, when the CPR counter is equal to zero, go to step (iii).

Step (ii)(f) may further comprise when the measured compression rate is less than the minimum required compression rate, when the CPR counter is not equal to zero, decrease the CPR counter by 1, produce a fifth feedback signal, wait for a plurality of compressions and go to step (ii)(b).

Alternatively, step (ii) may further comprise:
(ii)(a) set a CPR counter equal to x,
(ii)(b) receive an impedance signal of the subject measured during a plurality of CPR chest compressions by the rescuer,
(ii)(c) receive a compression rate measured during the plurality of CPR chest compressions,
(ii)(d) compare the measured compression rate with a maximum required compression rate,
(ii)(e) when the measured compression rate is less than the maximum required compression rate, go to step (iii),
(ii)(f) when the measured compression rate is greater than the maximum required compression rate, when the CPR counter is not equal to zero, decrease the CPR counter by 1, produce a sixth feedback signal and go to step (ii)(b),
(ii)(g) when the measured compression rate is greater than the maximum required compression rate, when the CPR counter is equal to zero, go to step (iii).

Step (ii)(f) may further comprise when the measured compression rate is greater than the maximum required compression rate, when the CPR counter is not equal to zero, decrease the CPR counter by 1, produce a sixth feedback signal, wait for a plurality of compressions and go to step (ii)(b).

Alternatively, step (ii) may further comprise:
(ii)(a) set a CPR counter equal to x,
(ii)(b) receive an impedance signal of the subject measured during a plurality of CPR chest compressions by the rescuer,
(ii)(c) receive a compression rate measured during the plurality of CPR chest compressions,
(ii)(d) compare the measured compression rate with a minimum required compression rate and a maximum required compression rate,
(ii)(e) when the measured compression rate is greater than the minimum required compression rate and when the measured compression rate is less than the maximum required compression rate, go to step (iii),
(ii)(f) when the measured compression rate is less than the minimum required compression rate, when the CPR counter is not equal to zero, decrease the CPR counter by 1, produce a fifth feedback signal and go to step (ii)(b),
(ii)(g) when the measured compression rate is less than the minimum required compression rate, when the CPR counter is equal to zero, go to step (iii), (ii)(h) when the measured compression rate is greater than the maximum required compression rate, when the CPR counter is not equal to zero, decrease the CPR counter by 1, produce a sixth feedback signal and go to step (ii)(b),
(ii)(i) when the measured compression rate is greater than the maximum required compression rate, when the CPR counter is equal to zero, go to step (iii).

Step (ii)(f) may further comprise when the measured compression rate is less than the minimum required compression rate, when the CPR counter is not equal to zero, decrease the CPR counter by 1, produce a fifth feedback signal, wait for a plurality of compressions and go to step (ii)(b).

Step (ii)(h) may further comprise when the measured compression rate is greater than the maximum required compression rate, when the CPR counter is not equal to zero, decrease the CPR counter by 1, produce a sixth feedback signal, wait for a plurality of compressions and go to step (ii)(b).

Step (ii) may further comprise:
(ii)(a) set a CPR counter equal to x,
(ii)(b) receive an impedance signal of the subject measured during a plurality of CPR chest compressions by the rescuer,
(ii)(c) receive a CPR chest compression depth measured during the plurality of CPR chest compressions,
(ii)(d) compare the measured compression depth with a minimum required compression depth,
(ii)(e) when the measured compression depth is greater than the minimum required compression depth, go to step (iii),
(ii)(f) when the measured compression depth is less than the minimum required compression depth, when the CPR counter is not equal to zero, decrease the CPR counter by 1, produce a seventh feedback signal and go to step (ii)(b),
(ii)(g) when the measured compression rate is less than the minimum required compression rate, when the CPR counter is equal to zero, go to step (iii).

Step (ii)(f) may further comprise when the measured compression depth is less than the minimum required compression depth, when the CPR counter is not equal to zero, decrease the CPR counter by 1, produce a seventh feedback signal, wait for a plurality of compressions and go to step (ii)(b).

Alternatively, step (ii) may further comprise:
(ii)(a) set a CPR counter equal to x,
(ii)(b) receive an impedance signal of the subject measured during a plurality of CPR chest compressions by the rescuer,
(ii)(c) receive a compression depth measured during the plurality of CPR chest compressions,
(ii)(d) compare the measured compression depth with a maximum required compression depth,
(ii)(e) when the measured compression depth is less than the maximum required compression depth, go to step (iii),
(ii)(f) when the measured compression depth is greater than the maximum required compression depth, when the CPR counter is not equal to zero, decrease the CPR counter by 1, produce an eighth feedback signal and go to step (ii)(b),
(ii)(g) when the measured compression depth is greater than the maximum required compression depth when the CPR counter is equal to zero, go to step (iii).

Step (ii)(f) may further comprise when the measured compression depth is greater than the maximum required compression depth, when the CPR counter is not equal to zero, decrease the CPR counter by 1, produce an eighth feedback signal, wait for a plurality of compressions and go to step (ii)(b).

Alternatively, step (ii) may further comprise:
(ii)(a) set a CPR counter equal to x,
(ii)(b) receive an impedance signal of the subject measured during a plurality of CPR chest compressions by the rescuer,
(ii)(c) receive a compression depth measured during the plurality of CPR chest compressions,
(ii)(d) compare the measured compression depth with a minimum required compression depth and a maximum required compression depth,
(ii)(e) when the measured compression depth is greater than the minimum required compression depth and when the measured compression depth is less than the maximum required compression depth, go to step (iii),
(ii)(f) when the measured compression depth is less than the minimum required compression depth, when the CPR counter is not equal to zero, decrease the CPR counter by 1, produce a seventh feedback signal and go to step (ii)(b),
(ii)(g) when the measured compression depth is less than the minimum required compression depth, when the CPR counter is equal to zero, go to step (iii), (ii)(h) when the measured compression depth is greater than the maximum required compression depth, when the CPR counter is not equal to zero, decrease the CPR counter by 1, produce an eighth feedback signal and go to step (ii)(b),
(ii)(i) when the measured compression depth is greater than the maximum required compression depth, when the CPR counter is equal to zero, go to step (iii).

Step (ii)(f) may further comprise when the measured compression depth is less than the minimum required compression depth, when the CPR counter is not equal to zero, decrease the CPR counter by 1, produce a seventh feedback signal, wait for a plurality of compressions and go to step (ii)(b).

Step (ii)(h) may further comprise when the measured compression depth is greater than the maximum required compression depth, when the CPR counter is not equal to zero, decrease the CPR counter by 1, produce an eighth feedback signal, wait for a plurality of compressions and go to step (ii)(b).

The feedback unit may receive the fifth feedback signal and issue CPR feedback in the form of a 'Push Faster' instruction to the rescuer. The feedback unit may receive the sixth feedback signal and issue CPR feedback in the form of a 'Push Slower' instruction to the rescuer. The feedback unit may receive the seventh feedback signal and issue CPR feedback in the form of a 'Push Harder' instruction to the rescuer. The feedback unit may receive the eighth feedback signal and issue CPR feedback in the form of a 'Push Softer' instruction to the rescuer.

## Claims

1. A defibrillator (20) configured to assess chest recoil of a subject during cardio pulmonary resuscitation (CPR) carried out by a rescuer on the subject and provide feedback to the rescuer, comprising:
a biosignal measurement system (22) configured to measure biosignals of the subject, determine when CPR is required and produce a CPR start signal and determine when CPR is to be ceased and produce a CPR stop signal,
an impedance measurement system (24) configured to measure impedance signals of the subject,
a CPR assessment system (26) connected to the biosignal measurement system (22) to receive the CPR start signal and the CPR stop signal, connected to the impedance measurement system (24) to receive impedance signals and configured to perform the steps:
(i) receive the CPR start signal and produce a first feedback signal,
(ii) receive an impedance signal of the subject measured during a plurality of CPR chest compressions by the rescuer,
(iii) use the impedance signal to assess chest recoil of the subject during the plurality of CPR chest compressions,
(iv) compare the chest recoil of the subject with a chest recoil threshold,
(v) when the chest recoil of the subject is greaterthan the chest recoil threshold, produce a second feedback signal and go to step (vii),
(vi) when the chest recoil of the subject is less than the chest recoil threshold, produce a third feedback signal and go to step (vii),
(vii) when the CPR stop signal is not received, return to step (ii),
(viii) when the CPR stop signal is received, produce a fourth feedback signal,
and
a feedback unit (28) connected to the CPR assessment system (26) and configured to receive the feedback signals and issue CPR feedback to the rescuer.

2. A defibrillator (20) according to claim 1 in which the CPR assessment system (26) is configured to use the impedance signal to assess chest recoil of the subject during the plurality of CPR chest compressions by measuring a characteristic of the impedance signal after at least some of the plurality of CPR chest compressions.

3. A defibrillator (20) according to claim 2 in which the CPR assessment system (26) is configured to use the impedance signal to assess chest recoil of the subject during the plurality of CPR chest compressions by measuring an amplitude of the impedance signal after at least some of the plurality of CPR chest compressions.

4. A defibrillator (20) according to claim 3 in which the CPR assessment system (26) is configured to compare the amplitude of the impedance signal after at least some of the plurality of CPR chest compressions with an impedance baseline of the subject.

5. A defibrillator (20) according to claim 4 in which the CPR assessment system (26) is configured to determine either incomplete chest recoil when the amplitude of the impedance signal after a CPR chest compression is not within a predetermined tolerance of the impedance baseline or complete chest recoil when the amplitude of the impedance signal after a CPR chest compression is within a predetermined tolerance of the impedance baseline.

6. A defibrillator (20) according to claim 5 in which the CPR assessment system (26) is configured to determine a proportion of incomplete chest recoils and compares the chest recoil of the subject with the chest recoil threshold by comparing the proportion of incomplete chest recoils with the chest recoil threshold.

7. A defibrillator (20) according to claim 6 in which the CPR assessment system (26) is configured to produce the second feedback signal when the proportion of incomplete chest recoils is greater than the chest recoil threshold and produce the third feedback signal when the proportion of incomplete chest recoils is less than the chest recoil threshold.

8. A defibrillator (20) according to any of claims 4 to 7 in which the CPR assessment system (26) is configured to receive an impedance signal of the subject measured during at least one period in which no CPR chest compressions are performed by the rescuer and use the impedance signal to establish the impedance baseline of the subject.

9. A defibrillator (20) according to claim 8 in which the CPR assessment system (26) is configured to receive an impedance signal of the subject measured during a first period before CPR chest compressions by the rescuer and use the impedance signal to establish a first impedance baseline and receive an impedance signal of the subject measured during one or more subsequent periods after CPR chest compressions by the rescuer and use the impedance signal to establish one or more subsequent impedance baselines.

10. A defibrillator (20) according to any preceding claim in which the impedance measurement system (24) is configured to measure the impedance signals of the subject by acquiring signals through electrodes of the defibrillator (20) placed on the chest of the subject.

11. A defibrillator (20) according to any preceding claim in which steps (v) to (vii) further comprise:
(v) when the chest recoil of the subject is greaterthan the chest recoil threshold, produce the second feedback signal and go to step (vii)(a),
(vi) when the chest recoil of the subject is less than the chest recoil threshold, produce the third feedback signal and go to step (vii)(b),
(vii)(a) when the CPR stop signal is not received, wait for a plurality of CPR chest compressions, return to step (ii),
(vii)(b) when the CPR stop signal is not received, return to step (ii).

12. A defibrillator (20) according to any preceding claim in which step (ii) further comprises:
(ii)(a) set a CPR counter equal to x,
(ii)(b) receive an impedance signal of the subject measured during a plurality of CPR chest compressions by the rescuer,
(ii)(c) receive a compression rate measured during the plurality of CPR chest compressions,
(ii)(d) compare the measured compression rate with a minimum required compression rate and a maximum required compression rate,
(ii)(e) when the measured compression rate is greater than the minimum required compression rate and when the measured compression rate is less than the maximum required compression rate, go to step (iii),
(ii)(f) when the measured compression rate is less than the minimum required compression rate, when the CPR counter is not equal to zero, decrease the CPR counter by 1, produce a fifth feedback signal and go to step (ii)(b),
(ii)(g) when the measured compression rate is less than the minimum required compression rate, when the CPR counter is equal to zero, go to step (iii),
(ii)(h) when the measured compression rate is greater than the maximum required compression rate, when the CPR counter is not equal to zero, decrease the CPR counter by 1, produce a sixth feedback signal and go to step (ii)(b),
(ii)(i) when the measured compression rate is greater than the maximum required compression rate, when the CPR counter is equal to zero, go to step (iii).

13. A defibrillator (20) according to any preceding claim in which step (ii) further comprises:
(ii)(a) set a CPR counter equal to x,
(ii)(b) receive an impedance signal of the subject measured during a plurality of CPR chest compressions by the rescuer,
(ii)(c) receive a compression depth measured during the plurality of CPR chest compressions,
(ii)(d) compare the measured compression depth with a minimum required compression depth and a maximum required compression depth,
(ii)(e) when the measured compression depth is greater than the minimum required compression depth and when the measured compression depth is less than the maximum required compression depth, go to step (iii),
(ii)(f) when the measured compression depth is less than the minimum required compression depth, when the CPR counter is not equal to zero, decrease the CPR counter by 1, produce a seventh feedback signal and go to step (ii)(b),
(ii)(g) when the measured compression depth is less than the minimum required compression depth, when the CPR counter is equal to zero, go to step (iii),
(ii)(h) when the measured compression depth is greater than the maximum required compression depth, when the CPR counter is not equal to zero, decrease the CPR counter by 1, produce an eighth feedback signal and go to step (ii)(b),
(ii)(i) when the measured compression depth is greater than the maximum required compression depth, when the CPR counter is equal to zero, go to step (iii).

14. A defibrillator (20) according to any preceding claim in which the feedback unit (28) is configured to receive the first feedback signal and issue CPR feedback in the form of a 'Start CPR and Push Hard' instruction to the rescuer, receive the second feedback signal and issue CPR feedback in the form of a 'Chest Recoil Incomplete' instruction to the rescuer, receive the third feedback signal and issue CPR feedback in the form of a 'Chest Recoil Good' instruction to the rescuer, receive the fourth feedback signal and issue CPR feedback in the form of a 'Stop CPR' instruction to the rescuer.

15. A defibrillator (20) according to claim 12 in which the feedback unit (28) is configured to receive the fifth feedback signal and issue CPR feedback in the form of a 'Push Faster' instruction to the rescuer, receive the sixth feedback signal and issue issues CPR feedback in the form of a 'Push Slower' instruction to the rescuer.

16. A defibrillator (20) according to claim 13 in which the feedback unit (28) is configured to receive the seventh feedback signal and issue CPR feedback in the form of a 'Push Harder' instruction to the rescuer, receive the eighth feedback signal and issue CPR feedback in the form of a 'Push Softer' instruction to the rescuer.

## Patentansprüche

1. Defibrillator (20), der dazu konfiguriert ist, eine Brustentlastung eines Subjekts während einer Herz-Lungen-Wiederbelebung (CPR), die durch einen Retter an dem Subjekt durchgeführt wird, zu beurteilen und dem Retter eine Rückmeldung bereitzustellen, umfassend:
ein Biosignalmesssystem (22), das dazu konfiguriert ist, Biosignale des Subjekts zu messen, zu bestimmen, wann eine CPR erforderlich ist, und ein CPR-Startsignal zu erzeugen und zu bestimmen, wann eine CPR zu beenden ist, und ein CPR-Stoppsignal zu erzeugen,
ein Impedanzmesssystem (24), das dazu konfiguriert ist, Impedanzsignale des Subjekts zu messen,
ein CPR-Beurteilungssystem (26), das mit dem Biosignalmesssystem (22) verbunden ist, um das CPR-Startsignal und das CPR-Stoppsignal zu empfangen, das mit dem Impedanzmesssystem (24) verbunden ist, um Impedanzsignale zu empfangen, und dazu konfiguriert ist, die folgenden Schritte auszuführen:
(i) Empfangen des CPR-Startsignals und Erzeugen eines ersten Rückmeldungssignals,
(ii) Empfangen eines Impedanzsignals des Subjekts, das während einer Vielzahl von CPR-Brustkompressionen durch den Retter gemessen wird,
(iii) Verwenden des Impedanzsignals, um eine Brustentlastung des Subjekts während der Vielzahl von CPR-Brustkompressionen zu beurteilen,
(iv) Vergleichen der Brustentlastung des Subjekts mit einer Brustentlastungsschwelle,
(v) wenn die Brustentlastung des Subjekts größer als die Brustentlastungsschwelle ist, Erzeugen eines zweiten Rückmeldungssignals und Fortfahren mit Schritt (vii),
(vi) wenn die Brustentlastung des Subjekts kleiner als die Brustentlastungsschwelle ist, Erzeugen eines dritten Rückmeldungssignals und Fortfahren mit Schritt (vii),
(vii) wenn das CPR-Stoppsignal nicht empfangen wird, Zurückkehren zu Schritt (ii),
(viii) wenn das CPR-Stoppsignal empfangen wird, Erzeugen eines vierten Rückmeldungssignals und
eine Rückmeldungseinheit (28), die mit dem CPR-Beurteilungssystem (26) verbunden und dazu konfiguriert ist, die Rückmeldungssignale zu empfangen und eine CPR-Rückmeldung an den Retter auszugeben.

2. Defibrillator (20) nach Anspruch 1, bei dem das CPR-Beurteilungssystem (26) dazu konfiguriert ist, das Impedanzsignal dazu zu verwenden, die Brustentlastung des Subjekts während der Vielzahl von CPR-Brustkompressionen zu beurteilen, indem es eine Eigenschaft des Impedanzsignals nach mindestens einigen der Vielzahl von CPR-Brustkompressionen misst.

3. Defibrillator (20) nach Anspruch 2, bei dem das CPR-Beurteilungssystem (26) dazu konfiguriert ist, das Impedanzsignal dazu zu verwenden, die Brustentlastung des Subjekts während der Vielzahl von CPR-Brustkompressionen zu beurteilen, indem eine Amplitude des Impedanzsignals nach mindestens einigen der Vielzahl von CPR-Brustkompressionen gemessen wird.

4. Defibrillator (20) nach Anspruch 3, bei dem das CPR-Beurteilungssystem (26) dazu konfiguriert ist, die Amplitude des Impedanzsignals nach mindestens einigen der Vielzahl von CPR-Brustkompressionen mit einer Impedanzbasislinie des Subjekts zu vergleichen.

5. Defibrillator (20) nach Anspruch 4, bei dem das CPR-Beurteilungssystem (26) dazu konfiguriert ist, entweder eine unvollständige Brustentlastung, wenn die Amplitude des Impedanzsignals nach einer CPR-Brustkompression nicht innerhalb einer vorbestimmten Toleranz der Impedanzbasislinie liegt, oder eine vollständige Brustentlastung zu bestimmen, wenn die Amplitude des Impedanzsignals nach einer CPR-Brustkompression innerhalb einer vorbestimmten Toleranz der Impedanzbasislinie liegt.

6. Defibrillator (20) nach Anspruch 5, bei dem das CPR-Beurteilungssystem (26) dazu konfiguriert ist, einen Anteil unvollständiger Brustentlastungen zu bestimmen, und die Brustentlastung des Subjekts mit der Brustentlastungsschwelle vergleicht, indem es den Anteil unvollständiger Brustentlastungen mit der Brustentlastungsschwelle vergleicht.

7. Defibrillator (20) nach Anspruch 6, bei dem das CPR-Beurteilungssystem (26) dazu konfiguriert ist, das zweite Rückmeldungssignal zu erzeugen, wenn der Anteil unvollständiger Brustentlastungen größer als die Brustentlastungsschwelle ist, und das dritte Rückmeldungssignal zu erzeugen, wenn der Anteil unvollständiger Brustentlastungen kleiner als die Brustentlastungsschwelle ist.

8. Defibrillator (20) nach einem der Ansprüche 4 bis 7, bei dem das CPR-Beurteilungssystem (26) dazu konfiguriert ist, ein Impedanzsignal des Subjekts zu empfangen, das während mindestens eines Zeitraums gemessen wird, in dem keine CPR-Brustkompressionen durch den Retter durchgeführt werden, und das Impedanzsignal dazu zu verwenden, die Impedanzbasislinie des Subjekts festzulegen.

9. Defibrillator (20) nach Anspruch 8, bei dem das CPR-Beurteilungssystem (26) dazu konfiguriert ist, ein Impedanzsignal des Subjekts zu empfangen, das während eines ersten Zeitraums vor CPR-Brustkompressionen durch den Retter gemessen wird, und das Impedanzsignal dazu zu verwenden, eine erste Impedanzbasislinie festzulegen, und ein Impedanzsignal des Subjekts zu empfangen, das während einer oder mehrerer aufeinanderfolgender Zeiträume nach CPR-Brustkompressionen durch den Retter gemessen wird, und das Impedanzsignal dazu zu verwenden, eine oder mehrere nachfolgende Impedanzbasislinien festzulegen.

10. Defibrillator (20) nach einem der vorhergehenden Ansprüche, bei dem das Impedanzmesssystem (24) dazu konfiguriert ist, die Impedanzsignale des Subjekts zu messen, indem es Signale durch Elektroden des Defibrillators (20) erfasst, die auf der Brust des Subjekts platziert sind.

11. Defibrillator (20) nach einem der vorhergehenden Ansprüche, bei dem die Schritte (v) bis (vii) ferner Folgendes umfassen:
(v) wenn die Brustentlastung des Subjekts größer als die Brustentlastungsschwelle ist, Erzeugen des zweiten Rückmeldungssignals und Fortfahren mit Schritt (vii)(a),
(vi) wenn die Brustentlastung des Subjekts kleiner als die Brustentlastungsschwelle ist, Erzeugen des dritten Rückmeldungssignals und Fortfahren mit Schritt (vii)(b),
(vii)(a) wenn das CPR-Stoppsignal nicht empfangen wird, Warten auf eine Vielzahl von CPR-Brustkompressionen, Zurückkehren zu Schritt (ii),
(vii)(b) wenn das CPR-Stoppsignal nicht empfangen wird, Zurückkehren zu Schritt (ii).

12. Defibrillator (20) nach einem der vorhergehenden Ansprüche, bei dem Schritt (ii) ferner Folgendes umfasst:
(ii)(a) Setzen eines CPR-Zählers gleich x,
(ii)(b) Empfangen eines Impedanzsignals des Subjekts, das während einer Vielzahl von CPR-Brustkompressionen durch den Retter gemessen wird,
(ii)(c) Empfangen einer Kompressionsrate, die während der Vielzahl von CPR-Brustkompressionen gemessen wird,
(ii)(d) Vergleichen der gemessenen Kompressionsrate mit einer minimal erforderlichen Kompressionsrate und einer maximal erforderlichen Kompressionsrate,
(ii)(e) wenn die gemessene Kompressionsrate größer als die minimal erforderliche Kompressionsrate ist und wenn die gemessene Kompressionsrate kleiner als die maximal erforderliche Kompressionsrate ist, Fortfahren mit Schritt (iii),
(ii)(f) wenn die gemessene Kompressionsrate kleiner als die minimal erforderliche Kompressionsrate ist, wenn der CPR-Zähler nicht gleich null ist, Verringern des CPR-Zählers um 1, Erzeugen eines fünften Rückmeldungssignals und Fortfahren mit Schritt (ii)(b),
(ii)(g) wenn die gemessene Kompressionsrate kleiner als die minimal erforderliche Kompressionsrate ist, wenn der CPR-Zähler gleich null ist, Fortfahren mit Schritt (iii),
(ii)(h) wenn die gemessene Kompressionsrate größer als die maximal erforderliche Kompressionsrate ist, wenn der CPR-Zähler nicht gleich null ist, Verringern des CPR-Zählers um 1, Erzeugen eines sechsten Rückmeldungssignals und Fortfahren mit Schritt (ii)(b),
(ii)(i) wenn die gemessene Kompressionsrate größer als die maximal erforderliche Kompressionsrate ist, wenn der CPR-Zähler gleich null ist, Fortfahren mit Schritt (iii).

13. Defibrillator (20) nach einem der vorhergehenden Ansprüche, bei dem Schritt (ii) ferner Folgendes umfasst:
(ii)(a) Setzen eines CPR-Zählers gleich x,
(ii)(b) Empfangen eines Impedanzsignals des Subjekts, das während einer Vielzahl von CPR-Brustkompressionen durch den Retter gemessen wird,
(ii)(c) Empfangen einer Kompressionstiefe, die während der Vielzahl von CPR-Brustkompressionen gemessen wird,
(ii)(d) Vergleichen der gemessene Kompressionstiefe mit einer minimal erforderlichen Kompressionstiefe und einer maximal erforderlichen Kompressionstiefe,
(ii)(e) wenn die gemessene Kompressionstiefe größer als die minimal erforderliche Kompressionstiefe ist und wenn die gemessene Kompressionstiefe kleiner als die maximal erforderliche Kompressionstiefe ist, Fortfahren mit Schritt (iii),
(ii)(f) wenn die gemessene Kompressionstiefe kleiner als die minimal erforderliche Kompressionstiefe ist, wenn der CPR-Zähler nicht gleich null ist, Verringern des CPR-Zählers um 1, Erzeugen eines siebten Rückmeldungssignals und Fortfahren mit Schritt (ii)(b),
(ii)(g) wenn die gemessene Kompressionstiefe kleiner als die minimal erforderliche Kompressionstiefe ist, wenn der CPR-Zähler gleich null ist, Fortfahren mit Schritt (iii),
(ii)(h) wenn die gemessene Kompressionstiefe größer als die maximal erforderliche Kompressionstiefe ist, wenn der CPR-Zähler nicht gleich null ist, Verringern des CPR-Zählers um 1, Erzeugen eines achten Rückmeldungssignals und Fortfahren mit Schritt (ii)(b),
(ii)(i) wenn die gemessene Kompressionstiefe größer als die maximal erforderliche Kompressionstiefe ist, wenn der CPR-Zähler gleich null ist, Fortfahren mit Schritt (iii).

14. Defibrillator (20) nach einem der vorhergehenden Ansprüche, bei dem die Rückmeldungseinheit (28) dazu konfiguriert ist, das erste Rückmeldungssignal zu empfangen und eine CPR-Rückmeldung in Form einer Anweisung "CPR starten und stark drücken" an den Retter auszugeben, das zweite Rückmeldungssignal zu empfangen und eine CPR-Rückmeldung in Form einer Anweisung "Brustentlastung unvollständig" an den Retter auszugeben, das dritte Rückmeldungssignal zu empfangen und eine CPR-Rückmeldung in Form einer Anweisung "Brustentlastung gut" an den Retter auszugeben, das vierte Rückmeldungssignal zu empfangen und eine CPR-Rückmeldung in Form einer Anweisung "CPR stoppen" an den Retter auszugeben.

15. Defibrillator (20) nach Anspruch 12, bei dem die Rückmeldungseinheit (28) dazu konfiguriert ist, das fünfte Rückmeldungssignal zu empfangen und eine CPR-Rückmeldung in Form einer Anweisung "schneller drücken" an den Retter auszugeben, das sechste Rückmeldungssignal zu empfangen und eine CPR-Rückmeldung in Form einer Anweisung "langsamer drücken" an den Retter auszugeben.

16. Defibrillator (20) nach Anspruch 13, bei dem die Rückmeldungseinheit (28) dazu konfiguriert ist, das siebte Rückmeldungssignal zu empfangen und eine CPR-Rückmeldung in Form einer Anweisung "stärker drücken" an den Retter auszugeben, das achte Rückmeldungssignal zu empfangen und eine CPR-Rückmeldung in Form einer Anweisung "schwächer drücken" an den Retter auszugeben.

## Revendications

1. Défibrillateur (20) conçu pour évaluer la détente thoracique d'un sujet lors d'une réanimation cardio-pulmonaire (RCP) effectuée par un secouriste sur le sujet et fournir une rétroaction au secouriste, comprenant :
un système de mesure de biosignaux (22) conçu pour mesurer les biosignaux du sujet, déterminer quand la RCP est requise et produire un signal de démarrage de RCP et déterminer quand la RCP doit être arrêtée et produire un signal d'arrêt de RCP,
un système de mesure d'impédance (24) conçu pour mesurer les signaux d'impédance du sujet,
un système d'évaluation de RCP (26) branché au système de mesure de biosignaux (22) destiné à recevoir le signal de démarage de RCP et le signal d'arrêt de RCP, branché au système de mesure d'impédance (24) destiné à recevoir des signaux d'impédance et conçu pour effectuer les étapes :
(i) réception du signal de démarrage de RCP et production d'un premier signal de rétroaction,
(ii) réception d'un signal d'impédance du sujet mesuré pendant une pluralité de compressions thoraciques de RCP par le secouriste,
(iii) utilisation du signal d'impédance pour évaluer la détente thoracique du sujet pendant la pluralité de compressions thoraciques de RCP,
(iv) comparaison de la détente thoracique du sujet à un seuil de détente thoracique,
(v) lorsque la détente thoracique du sujet est supérieure au seuil de détente thoracique, production d'un deuxième signal de rétroaction et passage à l'étape (vii),
(vi) lorsque la détente thoracique du sujet est inférieure au seuil de détente thoracique, production d'un troisième signal de rétroaction et passage à l'étape (vii),
(vii) lorsque le signal d'arrêt de RCP n'est pas reçu, retour à l'étape (ii),
(viii) lorsque le signal d'arrêt de RCP est reçu, production d'un quatrième signal de rétroaction, et
une unité de rétroaction (28) branchée au système d'évaluation de RCP (26) et conçue pour recevoir les signaux de rétroaction et émettre une rétroaction de RCP au secouriste.

2. Défibrillateur (20) selon la revendication 1, ledit système d'évaluation de RCP (26) étant conçu pour utiliser le signal d'impédance pour évaluer la détente thoracique du sujet pendant la pluralité de compressions thoraciques de RCP en mesurant une caractéristique du signal d'impédance après au moins une partie de la pluralité de compressions thoraciques de RCP.

3. Défibrillateur (20) selon la revendication 2, ledit système d'évaluation de RCP (26) étant conçu pour utiliser le signal d'impédance pour évaluer la détente thoracique du sujet pendant la pluralité de compressions thoraciques de RCP en mesurant l'amplitude du signal d'impédance après au moins une partie de la pluralité de compressions thoraciques de RCP.

4. Défibrillateur (20) selon la revendication 3, ledit système d'évaluation de RCP (26) étant conçu pour comparer l'amplitude du signal d'impédance après au moins une partie de la pluralité de compressions thoraciques de RCP à une ligne de base d'impédance du sujet.

5. Défibrillateur (20) selon la revendication 4, ledit système dévaluation de RCP (26) étant conçu pour déterminer soit une détente thoracique incomplète lorsque l'amplitude du signal d'impédance après une compression thoracique de RCP ne se trouve pas dans une tolérance prédéfinie de la ligne de base d'impédance, soit une détente thoracique complète lorsque l'amplitude du signal d'impédance après une compression thoracique de RCP se trouve dans une tolérance prédéfinie de la ligne de base d'impédance.

6. Défibrillateur (20) selon la revendication 5, ledit système d'évaluation de RCP (26) étant conçu pour déterminer une proportion de détentes thoraciques incomplètes et comparant la détente thoracique du sujet au seuil de détente thoracique en comparant la proportion de détentes thoraciques incomplètes au seuil de détente thoracique.

7. Défibrillateur (20) selon la revendication 6, ledit système d'évaluation de RCP (26) étant conçu pour produire le deuxième signal de rétroaction lorsque la proportion de détentes thoraciques incomplètes est supérieure au seuil de détente thoracique et produire le troisième signal de rétroaction lorsque la proportion de détentes thoraciques incomplètes est inférieure au seuil de détente thoracique.

8. Défibrillateur (20) selon l'une quelconque des revendications 4 à 7, ledit système d'évaluation de RCP (26) étant conçu pour recevoir un signal d'impédance du sujet mesuré pendant au moins une période pendant laquelle aucune compression thoracique de RCP n'est effectuée par le secouriste et utiliser le signal d'impédance pour établir la ligne de base d'impédance du sujet.

9. Défibrillateur (20) selon la revendication 8, ledit système d'évaluation de RCP (26) étant conçu pour recevoir un signal d'impédance du sujet mesuré pendant une première période avant les compressions thoraciques de RCP par le secouriste et utiliser le signal d'impédance pour établir une première ligne de base d'impédance et recevoir un signal d'impédance du sujet mesuré pendant une ou plusieurs périodes ultérieures après les compressions thoraciques de RCP par le secouriste et utiliser le signal d'impédance pour établir une ou plusieurs lignes de base d'impédance ultérieures.

10. Défibrillateur (20) selon l'une quelconque des revendications précédentes, ledit système de mesure d'impédance (24) étant conçu pour mesurer les signaux d'impédance du sujet en acquérant des signaux au moyen des électrodes du défibrillateur (20) placées sur le thorax du sujet.

11. Défibrillateur (20) selon l'une quelconque des revendications précédentes, lesdites étapes (v) à (vii) comprenant en outre :
(v) lorsque la détente thoracique du sujet est supérieure au seuil de compression thoracique, la production du deuxième signal de rétroaction et le passage à l'étape (vii)(a),
(vi) lorsque la détente thoracique du sujet est inférieure au seuil de détente thoracique, la production du troisième signal de rétroaction et le passage à l'étape (vii)(b),
(vii)(a) lorsque le signal d'arrêt de RCP n'est pas reçu, l'attente d'une pluralité de compressions thoraciques de RCP, le retour à l'étape (ii),
(vii)(b) lorsque le signal d'arrêt de RCP n'est pas reçu, le retour à l'étape (ii).

12. Défibrillateur (20) selon l'une quelconque des revendications précédentes, ladite étape (ii) comprenant en outre :
(ii)(a) le réglage d'un compteur de RCP égal à x,
(ii)(b) la réception d'un signal d'impédance du sujet mesuré pendant une pluralité de compressions thoraciques de RCP par le secouriste,
(ii)(c) la réception d'un taux de compression mesuré pendant la pluralité de compressions thoraciques de RCP,
(ii)(d) la comparaison du taux de compression mesuré à un taux de compression minimal requis et à un taux de compression maximal requis,
(ii)(e) lorsque le taux de compression mesuré est supérieur au taux de compression minimal requis et lorsque le taux de compression mesuré est inférieur au taux de compression maximal requis, le passage à l'étape (iii),
(ii)(f) lorsque le taux de compression mesuré est inférieur au taux de compression minimal requis, lorsque le compteur de RCP n'est pas égal à zéro, la diminution du compteur de RCP de 1, la production d'un cinquième signal de rétroaction et le passage à l'étape (ii)(b),
(ii)(g) lorsque le taux de compression mesuré est inférieur au taux de compression minimal requis, lorsque le compteur de RCP est égal à zéro, le passage à l'étape (iii),
(ii)(h) lorsque le taux de compression mesuré est supérieur au taux de compression maximal requis, lorsque le compteur de RCP n'est pas égal à zéro, la diminution du compteur de RCP de 1, la production d'un sixième signal de rétroaction et le passage à l'étape (ii)(b),
(ii)(i) lorsque le taux de compression mesuré est supérieur au taux de compression maximal requis, lorsque le compteur de RCP est égal à zéro, le passage à l'étape (iii).

13. Défibrillateur (20) selon l'une quelconque des revendications précédentes, ladite étape (ii) comprenant en outre :
(ii)(a) le réglage d'un compteur de RCP égal à x,
(ii)(b) la réception d'un signal d'impédance du sujet mesuré pendant une pluralité de compressions thoraciques de RCP par le secouriste,
(ii)(c) la réception d'une profondeur de compression mesurée pendant la pluralité de compressions thoraciques de RCP,
(ii)(d) la comparaison de la profondeur de compression mesurée à une profondeur de compression minimale requise et à une profondeur de compression maximale requise,
(ii)(e) lorsque la profondeur de compression mesurée est supérieure à la profondeur de compression minimale requise et lorsque la profondeur de compression mesurée est inférieure à la profondeur de compression maximale requise, le passage à l'étape (iii),
(ii)(f) lorsque la profondeur de compression mesurée est inférieure à la profondeur de compression minimale requise, lorsque le compteur de RCP n'est pas égal à zéro, la diminution du compteur de RCP de 1, la production d'un septième signal de rétroaction et le passage à l'étape (ii)(b),
(ii)(g) lorsque la profondeur de compression mesurée est inférieure à la profondeur de compression minimale requise, lorsque le compteur de RCP est égal à zéro, le passage à l'étape (iii),
(ii)(h) lorsque la profondeur de compression mesurée est supérieure à la profondeur de compression maximale requise, lorsque le compteur de RCP n'est pas égal à zéro, la diminution du compteur de RCP de 1, la production d'un huitième signal de rétroaction et le passage à l'étape (ii)(b),
(ii)(i) lorsque la profondeur de compression mesurée est supérieure à la profondeur de compression maximale requise, lorsque le compteur de RCP est égal à zéro, le passage à l'étape (iii).

14. Défibrillateur (20) selon l'une quelconque des revendications précédentes, ladite unité de rétroaction (28) étant conçue pour recevoir le premier signal de rétroaction et émettre une rétroaction de RCP sous la forme d'une instruction « Démarrer la RCP et appuyer fort » au secouriste, recevoir le deuxième signal de rétroaction et émettre une rétroaction de RCP sous la forme d'une instruction « Détente thoracique incomplète » au secouriste, recevoir le troisième signal de rétroaction et émettre une rétroaction de RCP sous la forme d'une instruction « Bonne détente thoracique » au secouriste, recevoir le quatrième signal de rétroaction et émettre la rétroaction de RCP sous la forme d'une instruction « Arrêter la RCP » au secouriste.

15. Défibrillateur (20) selon la revendication 12, ladite unité de rétroaction (28) étant conçue pour recevoir le cinquième signal de rétroaction et émettre une rétroaction de RCP sous la forme d'une instruction « Appuyer plus vite » au secouriste, recevoir le sixième signal de rétroaction et émettre une rétroaction de RCP sous la forme d'une instruction « Appuyer plus lentement » au secouriste.

16. Défibrillateur (20) selon la revendication 13, ladite unité de rétroaction (28) étant conçue pour recevoir le septième signal de rétroaction et émettre une rétroaction de RCP sous la forme d'un instruction « Appuyer plus fort » au secouriste, recevoir le huitième signal de rétroaction et émettre une rétroaction de RCP sous la forme d'une instruction « Appuyer plus doucement » au secouriste.
